# EUROPEAN PATENT SPECIFICATION

(11) **EP 3 202 395 B1**
(45) Date of publication and mention of the grant of the patent: **19.12.2018**
(21) Application number: 15847633.3
(22) Date of filing: 01.10.2015
(51) Int. Cl.: A61K 9/48, A61K 47/36, A61K 9/62, A61K 8/11, A23P 10/30, C08L 3/04, C08L 3/10, C08L 5/00, A61Q 19/00, A61K 8/73

(54) **SOFT CAPSULE SHELL**
WEICHKAPSELHÜLLE
ENVELOPPE DE CAPSULE MOLLE

(30) Priority: 03.10.2014 JP 2014204561
(43) Date of publication of application: 09.08.2017
(73) Proprietor: Fuji Capsule Co., Ltd., Fujinomiya-shi, Shizuoka 418-0112 (JP)
(72) Inventor: WATANABE, Kazuhiko, Fujinomiya-shi Shizuoka 418-0112 (JP); KONDO, Yosuke, Fujinomiya-shi Shizuoka 418-0112 (JP); SHIMOKAWA, Yoshiyuki, Fujinomiya-shi Shizuoka 418-0112 (JP); KATO, Kenji, Fujinomiya-shi Shizuoka 418-0112 (JP)
(74) Representative: Blodig, Wolfgang
(86) International application number: PCT/JP2015/077926
(87) International publication number: WO 2016/052699

(56) References cited:
- JP-A- 2001 275 585
- JP-A- 2011 026 262
- JP-A- 2011 026 262
- JP-A- 2013 040 217
- JP-A- 2013 040 217
- JP-A- 2014 015 431
- JP-A- 2015 189 684
- US-A1- 2002 081 331
- R. HOOVER ET AL: "The Effect of Heat-Moisture Treatment on the Structure and Physicochemical Properties of Normal Maize, Waxy Maize, Dull Waxy Maize and Amylomaize V Starches", JOURNAL OF CEREAL SCIENCE., vol. 23, no. 2, March 1996 (1996-03), pages 153-162, XP055463126, GB ISSN: 0733-5210, DOI: 10.1006/jcrs.1996.0015
- HOLMES, MALCOLM GLENNIE ET AL.: 'STUDIES ON BARLEY AND MALT WITH THE RAPID VISCOANALYSER: [I] THE EFFECT OF VARIATIONS IN PHYSICAL AND CHEMICAL PARAMETERS' J. INST. BREW. vol. 101, 1995, pages 11 - 18, XP055424725

## Description

### Technical Field

The present invention relates to a soft capsule shell, a soft capsule produced therewith, and a method for producing a soft capsule.

### Background Art

Soft capsules are used in a wide range of fields including pharmaceuticals, foods, and cosmetics. The most popular shell base material for the soft capsules is gelatin. Gelatin is inexpensive and non-toxic and has good mechanical strength and good shell-forming ability.

On the other hand, the recent issue of bovine spongiform encephalopathy (BSE) and religious reasons have created a demand for an alternative to animal-derived gelatin, and capsules are no exception.

Thus, a variety of studies have been conducted on soft capsule shells produced with plant-derived base materials, and there have been previously reported, for example, a soft capsule shell including modified starch, which is obtained by subjecting glutinous corn starch to an acid treatment or a heat-moisture treatment in the presence of a salt, and 20 to 28 parts by weight of iota carrageenan based on 100 parts by weight of the modified starch (Patent Literature 1), and a film-forming composition for soft capsule including acid-degraded waxy corn starch, a gelling agent, and a plasticizer (Patent Literature 2).

### Citation List

### Patent Literatures

Patent Literature 1: JP 2011-26262 A
Patent Literature 2: JP 2010-180159 A

### Summary of Invention

### Technical Problem

In general, soft capsule shells are required to satisfy the following requirements during capsule production: appropriate fluidity of a shell solution, good film-forming ability, an appropriate sheet shell strength, elongation and adhesive property, and the like. In addition, the capsule after encapsulation is required to satisfy, for example, blocking resistance enough to prevent capsules from attaching to each other, elasticity, transparency, and disintegrability.

In fact, however, it is difficult to say that the conventional techniques totally satisfy such shell performances. In particular, shell sheets produced from the conventional shell composition and having the same shell sheet thickness as that of common gelatin shell (about 0.9 mm) are often insufficient in strength or adhesive force. Therefore, the thickness of the shell sheets was reduced (to about 0.6 mm) in order to ensure a necessary level of shell sheet strength and adhesive force for encapsulation. However, when the shell sheets are thinned, a problem sometimes occurs in that when particles in the content liquid are trapped in a part of a capsule where the shells are bonded, very small holes are formed in the bonded part to allow the content of the capsule to leak out. This problem becomes significant as the shell sheet thickness decreases.

The present invention, which has been accomplished in view of the circumstances described above, is directed to providing a plant-derived soft capsule shell having high shell sheet strength, good adhesive property, and good shell performances, and also directed to providing a soft capsule.

### Solution to Problem

As a result of various studies to solve the problems, the inventors have found that using a combination of iota carrageenan and waxy corn starch having undergone a heat-moisture treatment in the presence of a salt, in a specific ratio range, and using a combination of a carrageenan material including iota carrageenan and a starch material including the waxy corn starch, in a specific ratio range, make it possible to obtain a capsule shell that can provide high shell sheet strength and prevent a shell base material from being trapped in the bonded part so that good adhesive property can be achieved. The inventors have also found that using such combinations also makes it possible to obtain good shell performances such as good blocking resistance, elasticity, and transparency, and rapid disintegrability after encapsulation. The present invention has been completed based on the findings.

That is, the present invention provides a soft capsule shell comprising the following components (A) and (B):
(A) iota carrageenan; and
(B) waxy corn starch having undergone a heat-moisture treatment in the presence of a salt, wherein the content mass ratio [(A)/(B)] of the component (A) to the component (B) is from 0.4 to 3, and the content mass ratio [carrageenan/starch] of carrageenan including the component (A) to starch including the component (B) is from 0.29 to 1.

The present invention also provides a soft capsule obtained by using the soft capsule shell.

### Advantageous Effects of Invention

According to the present invention, soft capsules resistant to leakage of contents can be obtained without using animal-derived gelatin. The soft capsule of the present invention also has good shell performances such as good blocking resistance, good elasticity, good transparency, and good disintegrability after drying.

### Description of Embodiments

The soft capsule shell of the present invention comprises (A) iota carrageenan and (B) waxy corn starch having undergone a heat-moisture treatment in the presence of a salt, wherein the content mass ratio [(A)/(B)] of the component (A) to the component (B) is from 0.4 to 3, and the content mass ratio [carrageenan/starch] of carrageenan including the component (A) to starch including the component (B) is from 0.29 to 1.

Carrageenan is a kind of galactan having sulfate groups and is known to be present in red algae. According to the regulation of food additives in Japan, there are defined three types: "purified carrageenan," "processed Eucheuma seaweed," and "Eucheuma seaweed powder" (see Annotated Book of the List of Existing Food Additives (1999), published by Japan Food Additives Association). They differ only in degree of purification, and all of them are substantially encompassed by the term "carrageenan" in the present invention.

The carrageenan may be a pure product or may contain a standardizing agent. In this regard, the standardizing agent may be one or more selected from the group consisting of saccharides such as sucrose, glucose, maltose, and lactose, and dextrin. When the carrageenan contains the standardizing agent, the content of the standardizing agent is preferably 50% by mass or less, more preferably 45% by mass or less. In this regard, when the carrageenan used contains the standardizing agent, the content of the standardizing agent is counted as part of the carrageenan content.

Carrageenan may be mainly classified into three types : iota carrageenan, kappa carrageenan, and lambda carrageenan according to the difference in gelation properties or structure. In the present invention, iota carrageenan is used as the component (A).

In view of shell sheet strength or elasticity, the content of iota carrageenan (A) is preferably from 20 to 50% by mass, more preferably from 25 to 48% by mass, even more preferably from 25 to 45% by mass, further more preferably from 25 to 40% by mass, based on the total amount of the solid components. As used herein, the term "solid components" refers to all components of the shell composition except for purified water and a plasticizer.

For gelation enhancement, the soft capsule shell of the present invention preferably further contains kappa carrageenan.

In view of gelation strength, the content of kappa carrageenan is preferably from 0.05 to 3.5% by mass, more preferably from 0.1 to 3% by mass, even more preferably from 0.1 to 2.5% by mass, based on the total amount of the solid components.

Waxy corn starch having undergone a heat-moisture treatment in the presence of a salt (B) to be used in the present invention can be obtained by heating waxy corn starch under wet conditions in the presence of a salt. The component (B) can be produced by performing the heat-moisture treatment, then drying, and optionally grinding.

Such starch is known and produced from waxy corn starch by, for example, the method described in JP 4608051 B1 or methods similar thereto.

A starch paste solution prepared from the waxy corn starch having undergone a heat-moisture treatment in the presence of a salt (B) preferably has a viscosity of 100 to 3,000 mPa·s (B-type viscometer, starch content 40% by mass, liquid temperature 90°C). Preferably, when the viscosity is 100 mPa·s or more, the starch can create a "thick feel" and form a flexible shell sheet, which can be easily elongated and easilyprovide good adhesive property. Preferably, when the viscosity is 3,000 mPa·s or less, the starch can be easily kneaded, and a strong shell sheet can be easily formed through the increased gelation of carrageenan.

The waxy corn starch to be subjected to the heat-moisture treatment is preferably not chemically modified, in other words, preferably does not have undergone any chemical modification such as acid treatment, gelatinization, etherification, or acetylation.

In view of adhesive property, the content of the waxy corn starch having undergone a heat -moisture treatment in the presence of a salt (B) is preferably from 5 to 75% by mass, more preferably from 30 to 45% by mass, based on the total amount of the solid components.

In the present invention, the content mass ratio [(A)/(B)] of the iota carrageenan (A) to the waxy corn starch having undergone a heat-moisture treatment in the presence of a salt (B) is from 0.4 to 3. From comprehensive perspectives (including shell strength and gelation strength), the content mass ratio [(A)/(B)] is more preferably from 0.6 to 1.2.

If necessary, the soft capsule shell of the present invention may contain one or more kinds of starch such as unmodified starch, modified starch, and starch degradation products.

Any of these starches may be added to such an extent that the content mass ratio [carrageenan/starch] of the carrageenan including the iota carrageenan (A) to the starch including the waxy corn starch having undergone a heat-moisture treatment in the presence of a salt (B) falls within the range of 0.29 to 1. That is, the starch used to form the soft capsule shell of the present invention may be only the waxy corn starch having undergone a heat-moisture treatment in the presence of a salt (B), or may be a combination of the waxy corn starch having undergone a heat-moisture treatment in the presence of a salt (B) and one or more kinds of starch other than the waxy corn starch (B).

In particular, from comprehensive perspectives (including shell strength and gelation strength), the waxy corn starch having undergone a heat-moisture treatment in the presence of a salt (B) is preferably used in combination with one or more kinds of starch other than the waxy corn starch (B). Oxidized starch and waxy potato starch are preferred starch other than the waxy corn starch (B).

The waxy potato starch is preferably starch obtained by acid treatment of waxy potato starch. From comprehensive perspectives (including shell strength and gelation strength), a starch paste solution prepared from the waxy potato starch preferably has a viscosity of 6 mPa·s or more, more preferably 10 to 20 mPa·s (starch content 30% by mass, liquid temperature 80°C).

In the present invention, the content mass ratio [carrageenan/starch] of the carrageenan to the starch is from 0.29 to 1. From comprehensive perspectives (including shell strength, elasticity, gelation strength), the content mass ratio [carrageenan/starch] is more preferably from 0.4 to 0.7.

If necessary, the soft capsule shell of the present invention may contain a gelling agent other than iota or kappa carrageenan, such as sodium alginate, pullulan, glucomannan, gum arabic, or furcellaran.

Inviewof the physical properties of the shell and the quality of the soft capsule, the content of the gelling agent other than iota or kappa carrageenan is preferably 1% by mass or less, more preferably 0.5% by mass or less, even more preferably 0.1% by mass or less, based on the total amount of the solid components.

If necessary, the soft capsule shell of the present invention may also contain any of various additives used for soft capsule shells, such as plasticizers, natural colorants, synthetic colorants, various sweeteners, preservatives, water activity-reducing agents, and pH adjusters.

Examples of plasticizers include, but are not limited to, glycerin, sorbitol, erythritol, propylene glycol, and polyethylene glycol. Among them, glycerin and sorbitol are preferred.

In view of flexibility, the content of the plasticizer in the soft capsule shell of the present invention is preferably 30 parts by mass or more, more preferably 35 parts by mass or more, based on 100 parts by mass of the total amount of the solid components. In view of stickiness, the content of the plasticizer is preferably 60 parts by mass or less, more preferably 50 parts by mass or less, based on 100 parts by mass of the total amount of the solid components. The content of the plasticizer is preferably from 30 to 60 parts by mass, more preferably from 35 to 50 parts by mass, based on 100 parts by mass of the total amount of the solid components.

The soft capsule shell of the present invention may be produced by a conventional method. For example, the soft capsule shell of the present invention may be produced by a process that includes stirring and dispersing the iota carrageenan (A), the waxy corn starch having undergone a heat-moisture treatment in the presence of a salt (B), and optionally any of various additives in water, stirring and solving them at 70 to 98°C, and then defoaming the solution under vacuum.

The amount of water is preferably 100 parts by mass or more, more preferably 150 parts by mass or more, based on 100 parts by mass of the total amount of the solid components. When the amount of water in the shell solution is relatively large, the shell obtainedafter drying will be rather thin, which canproduce effects such as easy swallowing and rapid disintegration.

Soft capsules can be obtained by forming the resulting soft capsule shell into capsules with a specific shape and drying the capsules. The soft capsules may be produced by a method conventionally used for producing soft capsules, such as a plate method or a punching method using a rotary die soft capsule filling machine or the like.

In particular, the soft capsules are preferably produced by a rotary die process in view of industrial productivity and effective achievement of the advantageous effects of the present invention. A rotary die soft capsule filling machine is mainly composed of rotary drums, a pair of rotary dies (die rolls), and segments, which is used in a method including casting a soft capsule shell solution on the rotary drums to form two shell sheets and punching the shell sheets into capsules by using the pair of rotary die (die roll), to thereby perform forming soft capsules and filling the capsules with a content simultaneously.

Generally, in a rotary die soft capsule filling machine, a soft capsule shell solution is cast on a pair of cooled rotary drums to form a pair of sheets, which are subjected to a capsule formation process that includes heating the pair of shell sheets to a temperature necessary for sealing (generally 40 °C or higher) by the segments arranged above the pair of rotary dies (die rolls), so that soft capsules completely sealed are obtained by the pressure provided by the die rolls and heat sealing (see, for example, Patent Literature 1).

However, when exposed to high temperature, the content of capsules may undergo denaturation during the heat sealing for encapsulation. In a preferred mode of the present invention, therefore, a pair of shell sheets are heated to a temperature higher than room temperature, for example, 30 to 80°C, preferably 40 to 75°C, and then fed between a pair of rotary dies (die rolls), in which the shell sheets are pressure-sealed and formed into capsules while the segments are kept at room temperature (22 to 28°C) without being heated. Heating the shell sheets in advance makes it possible to improve the mechanical strength of the shell sheets and to improve the adhesive property of the shell sheets during the pressure sealing, so that soft capsules with higher quality can be formed. In addition, avoiding heat sealing makes it possible to protect the content of capsules from heating, which enables encapsulation of, for example, less heat-resistant drugs.

The heating of the shell sheets may be performed by, for example, a method of heating the rotary drums to 30 to 80°C, preferably 40 to 75°C, or a method of heating them using heating means such as an infrared heater or blowing hot air between the rotary drum and the die. In this case, the shell sheets may be heated to a temperature higher than room temperature after they are formed until they reach the rotary dies, and also the shell sheets may have a temperature lowered to room temperature when they reach the rotary dies.

Examples of the shape of the soft capsule include, but are not limited to, an oval (football) shape, an oblong (extended elliptical) shape, a round (spherical) shape, a tube shape, and a special shape such as a self-cut shape. In this regard, the "self-cut shape" includes an encapsulated hollow body and a tab connected to the top end of the body via a neck portion. When it is used, the tab is twisted off from the body so that a drug, a cosmetic, a food, a chemical, or any other content encapsulated in the body is allowed to flow out. In some cases, it is also called a "twist-off" or "wrench-off" shape.

The soft capsule of the present invention can be used in a variety of applications such as pharmaceuticals, quasi-drugs, cosmetics, and foods. The composition of the content of the capsule maybe appropriate ly determined depending on the intended use. The content maybe in a form of solution, suspension, paste, powder, or granules, or any other form.

Hereinafter, examples of components that may be contained in the capsule will be shown. Any of such components may be contained in any part of the capsule.

Examples of oils and fats that maybe contained in the capsule include avocado oil, almond oil, linseed oil, fennel oil, perilla oil, olive oil, olive squalene, orange oil, orange roughy oil, sesame oil, garlic oil, cacao butter, pumpkin seed oil, chamomile oil, carrot oil, cucumber oil, beef tallow fatty acid, kukui nut oil, cranberry seed oil, brown rice germ oil, rice oil, wheat germ oil, safflower oil, shea butter, liquid shea butter, perilla oil, soybean oil, evening primrose oil, camellia oil, corn oil, rapeseed oil, saw palmetto extract oil, Job's tears seed oil, persic oil, parsley seed oil, castor oil, sunflower oil, grape seed oil, borage oil, macadamia nut oil, meadowfoam oil, cottonseed oil, peanut oil, turtle oil, mink oil, egg-yolk oil, fish oil, palm oil, palm kernel oil, Japan wax, coconut oil, long-chain, medium-chain, and short-chain fatty acid triglycerides, diacylglycerides, beef tallow, lard, squalene, squalane, pristane, and hydrogenated products of these oils and fats.

Examples of waxes that maybe contained in the capsule include shellac wax, beeswax, carnauba wax, spermaceti, lanolin, liquid lanolin, reduced lanolin, hard lanolin, cyclic lanolin, lanolin wax, candelilla wax, Japan wax, montan wax, shellac wax, and rice wax. Examples of hardened oils that may be contained in the capsule include hardened vegetable oils obtained by hydrogenating vegetable oils and fats, hardened beef tallow, and hardened lard.

Examples of mineral oils that may be contained in the capsule include liquid paraffin, petrolatum, paraffin, ozokerite, ceresin, and microcrystalline wax.

Examples of fatty acids that may be contained in the capsule include natural fatty acids such as lauric acid, myristic acid, palmitic acid, stearic acid, behenic acid, oleic acid, linoleic acid, conjugated linoleic acid, linolenic acid, docosahexaenoic acid, eicosapentaenoic acid, 12-hydroxystearic acid, undecylenic acid, tall oil, and lanolin fatty acid; synthetic fatty acids such as isononanoic acid, caproic acid, 2-ethylbutanoic acid, isopentanoic acid, 2-methylpentanoic acid, 2-ethylhexanoic acid, and isopentanoic acid; and oils and fats containing any of these fatty acids as fatty acid components.

Examples of vitamins that may be contained in the capsule include A-group vitamins such as retinol, retinal (vitamin A1), dehydroretinal (vitaminA2), carotene, and lycopene (provitamin A); B-group vitamins such as fursultiamine, thiamine hydrochloride, and thiamine sulfate (vitamin B1), riboflavin (vitamin B2), pyridoxine (vitamin B6), cyanocobalamin, methylcobalamin (vitamin B12), folic acids, nicotinic acids, pantothenic acids, biotins, choline, and an inositol; C-group vitamins such as ascorbic acid or derivatives thereof; D-group vitamins such as ergocalciferol (vitamin D2), cholecalciferol (vitamin D3), and dihydrotachysterol; E-group vitamins such as vitamin E or derivatives thereof; ubiquinones; K-group vitamins such as phytonadione (vitamin K1), menaquinone (vitamin K2), menatetrenone, menadione (vitamin K3), and menadiol (vitamin K4), and others such as essential fatty acid (vitamin F), carnitine, ferulic acid, γ-oryzanol, orotic acid, P-group vitamins (rutin, eriocitrin, and hesperidin), and vitamin U.

Examples of stimulants that may be contained in the capsule include capsicum tincture, capsicum oil, nonylic acid vanillylamide, cantharides tincture, ginger tincture, ginger oil, mint oil, 1-menthol, camphor, and benzyl nicotinate.

Examples of ultraviolet absorbing or shielding agents that maybe contained in the capsule include benzophenone derivatives (such as 2-hydroxy-4-methoxybenzophenone, 2-hydroxy-4-methoxybenzophenone-5-sulfonic acid, sodium 2-hydroxy-4-methoxybenzophenone-5-sulfonate, dihydroxydimethoxybenzophenone, sodium dihydroxydimethoxybenzophenone-sulfonate, 2,4-dihydroxybenzophenone, and tetrahydroxybenzophenone), p-aminobenzoic acid derivatives (such as p-aminobenzoic acid, ethyl p-aminobenzoate, glyceryl p-aminobenzoate, amyl p-dimethylaminobenzoate, and octyl p-dimethylaminobenzoate), methoxycinnamic acid derivatives (such as ethyl p-methoxycinnamate, isopropyl p-methoxycinnamate, octyl p-methoxycinnamate, 2-ethoxyethyl p-methoxycinnamate, sodium p-methoxycinnamate, potassiump-methoxycinnamate, and glyceryl di-p-methoxycinnamate mono-2-ethylhexanoate), salicylic acid derivatives (such as octyl salicylate, phenyl salicylate, homomenthyl salicylate, dipropylene glycol salicylate, ethylene glycol salicylate, myristyl salicylate, and methyl salicylate), anthranilic acid derivatives (such as methyl anthranilate), urocanic acid derivatives (such as urocanic acid and ethyl urocanate), coumarin derivatives, amino acid compounds, benzotriazole derivatives, tetrazole derivatives, imidazoline derivatives, pyrimidine derivatives, dioxane derivatives, camphor derivatives, furan derivatives, pyrone derivatives, nucleic acid derivatives, allantoin derivatives, nicotinic acid derivatives, vitamin B6 derivatives, umbelliferone, esculin, benzyl cinnamate, cinoxate, oxybenzone, dioxybenzone, octabenzone, sulisobenzone, benzoresorcinol, arbutin, guaiazulene, shikonin, baicalin, baicalein, berberine, Neo Heliopan, Escalol, zinc oxide, talc, and kaolin.

Examples of whitening agents that may contained in the capsule include p-aminobenzoic acid derivatives, salicylic acid derivatives, anthranilic acid derivatives, coumarin derivatives, amino acid compounds, benzotriazole derivatives, tetrazole derivatives, imidazoline derivatives, pyrimidine derivatives, dioxane derivatives, camphor derivatives, furan derivatives, pyrone derivatives, nucleic acid derivatives, allantoin derivatives, nicotinic acid derivatives, vitamin C or derivatives thereof (such as vitamin C phosphate magnesium and vitamin C glucoside), vitamin E or derivatives thereof, kojic acid or derivatives thereof, oxybenzone, benzophenone, arbutin, guaiazulene, shikonin, baicalin, baicalein, berberine, placenta extract, ellagic acid, and rucinol.

Examples of tyrosinase activity inhibitors that may be contained in the capsule include vitamin C or derivatives thereof (such as vitamin C phosphate magnesium and vitamin C glucoside), hydroquinone or derivatives thereof (such as hydroquinone benzyl ether), kojic acid or derivatives thereof, vitamin E or derivatives thereof, N-acetyltyrosine or derivatives thereof, glutathione, hydrogen peroxide, zinc peroxide, placenta extract, ellagic acid, arbutin, rucinol, silk extract, and plant extracts (chamomile, mulberry, gardenia, Japanese angelica root, burnet, shrubby sophora, mugwort, Japanese honeysuckle, phellodendron bark, Houttuynia cordata, Wolfiporia extensa, Job's tears, white dead nettle, hop, crataegus fruit, eucalyptus, yarrow, Althaea Officinalis, cinnamon, vitex rotundifolia fruit, hamamelis, white mulberry or Japanese mulberry, isodon herb, platycodon root, dodder seed, Euphorbia lathyris seed, leopard flower, ephedra herb, cnidium rhizome, aralia rhizome, bupleurum root, Saposhnikovia, American silvertop, scutellaria root, moutan bark, peony root, Geranium, pueraria root, glycyrrhiza, sumac gallnut, aloe, cimicifuga rhizome, safflower, green tea, black tea, and gambir).

Examples of melanin pigment reducing or decomposing substances that may be contained in the capsule include phenylmercuric hexachlorophene, mercuric oxide, mercurous chloride, hydrogen peroxide water, zinc peroxide, and hydroquinone or derivatives thereof.

Examples of substances for accelerating turnover or activating cells that may be contained in the capsule include hydroquinone, lactic acid bacteria extract, placenta extract, reishi mushroom extract, vitamin A, vitamin E, allantoin, spleen extract, thymus extract, yeast extract, fermented milk extract, and plant extracts (aloe, scutellaria root, horsetail, gentian, burdock, lithospermum root, carrot, hamamelis, hop, coix seed, white dead nettle, Swertia japonica, Japanese angelica root, pot marigold, sweet hydrangea leaf, Hypericum erectum, cucumber, thyme, rosemary, and parsley).

Examples of astringents that may be contained in the capsule include succinic acid, allantoin, zinc chloride, zinc sulfate, zinc oxide, calamine, zinc p-phenolsulfonate, aluminum potassium sulfate, resorcin, ferric chloride, and tannic acids (including cathechin compounds).

Examples of active oxygen scavengers that may be contained in the capsule include SOD, catalase, and glutathione peroxidase.

Examples of antioxidants that maybe contained in the capsule include vitamin C or salts thereof, stearic acid esters, vitamin E or derivatives thereof, nordihydroguaiaretic acid, butylhydroxytoluene (BHT), butylhydroxyanisole (BHA), hydroxytyrosol, p-hydroxyanisole, propyl gallate, sesamol, sesamolin, gossypol, and propolis.

Examples of lipid peroxide formation inhibitors that may be contained in the capsule include β-carotene and plant extracts (such as cultured sesame cells, sweet hydrangea leaf, Hypericum erectum, hamamelis, clove, melissa, isodon herb, white birch, scarlet sage, rosemary, Nandina fruit, rose fruit, ginkgo, and green tea).

Examples of anti-inflammatory agents that may be contained in the capsule include ichthammol, indomethacin, kaolin, salicylic acid, sodium salicylate, methyl salicylate, acetylsalicylic acid, diphenhydramine hydrochloride, d-camphor, dl-camphor, hydrocortisone, guaiazulene, chamazulene, chlorpheniramine maleate, glycyrrhizic acid or salts thereof, glycyrrhetinic acid or salts thereof, glycyrrhiza extract, lithospermum root extract, rose fruit extract, and propolis.

Examples of antimicrobials, microbicides, or disinfectants that may be contained in the capsule include acrinol, sulfur, calcium gluconate, chlorhexidine gluconate, sulfamine, mercurochrome, lactoferrin or hydrolysates thereof, alkyldiaminoethylglycine chloride solutions, triclosan, sodium hypochlorite, chloramine T, bleaching powder, iodine compounds, iodoform, sorbic acid or salts thereof, propionic acid or salts thereof, salicylic acid, dehydroacetic acid, p-hydroxybenzoic acid esters, undecylenic acid, thiamine lauryl sulfate, thiamine lauryl nitrate, phenol, cresol, p-chlorophenol, p-chloro-m-xylenol, p-chloro-m-cresol, thymol, phenethyl alcohol, O-phenylphenol, Irgasan CH3565, halocarban, hexachlorophene, chlorhexidine, ethanol, methanol, isopropyl alcohol, benzyl alcohol, ethylene glycol, propylene glycol, 2-phenoxyethanol, 1,2-pentanediol, zinc pyrithione, chlorobutanol, isopropylmethylphenol, nonionic surfactants (such as polyoxyethylene lauryl ether, polyoxyethylene nonyl phenyl ether, and polyoxyethylene octyl phenyl ether), amphoteric surfactants, anionic surfactants (such as sodium lauryl sulfate and potassium lauroyl sarcosinate), cationic surfactants (such as cetyltrimethylammonium bromide, benzalkonium chloride, benzethonium chloride, and methylrosaniline chloride), formaldehyde, hexamine, brilliant green, malachite green, crystal violet, Germall, photosensitizer 101, photosensitizer 201, photosensitizer 401, N-long chain acyl basic amino acid derivatives and acid addition salts thereof, zinc oxide, hinokitiol, sophora root, and propolis.

Examples of moisturizing agents that may be contained in the capsule include glycerin, propylene glycol, 1,3-butylene glycol, polyethylene glycol, caprylic/capric triglyceride, glycolicacid (α-hydroxyacid), hyaluronic acid or salts thereof, chondroitin sulfate or salts thereof, water-soluble chitin or derivatives thereof, chitosan derivatives, pyrrolidonecarboxylic acid or salts thereof, sodium lactate, urea, sorbitol, amino acids or derivatives thereof (valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, histidine, sulfates thereof, phosphates thereof, nitrates thereof, citrates thereof, or pyrrolidonecarboxylic acid).

Examples of various organic acids that may be contained in the capsule include glycolic acid, citric acid, malic acid, tartaric acid, lactic acid, ferulic acid, and phytic acid.

Examples of agents for hair that may be contained in the capsule include selenium disulfide, alkylisoquinolium bromide solutions, zinc pyrithione, biphenamine, thianthol, castoreum tincture, ginger tincture, capsicum tincture, quinine hydrochloride, strong ammonia water, potassium bromate, sodium bromate, and thioglycolic acid.

Examples of perfumes that may be contained in the capsule include natural animal perfumes such as musk, civet, castoreum, and ambergris; plant perfumes such as anise essential oil, angelica essential oil, ylangylang essential oil, iris essential oil, fennel essential oil, orange essential oil, cananga essential oil, caraway essential oil, cardamon essential oil, guaiacwood essential oil, cumin essential oil, Kuromoji (Lindera umbellata) essential oil, cinnamon bark essential oil, cinnamon essential oil, geranium essential oil, copaibabalsam essential oil, coriander essential oil, perilla essential oil, cedar wood essential oil, citronella essential oil, jasmine essential oil, gingergrass essential oil, Japanese cedar essential oil, spearmint essential oil, peppermint essential oil, star anise essential oil, tuberose essential oil, clove essential oil, neroli essential oil, wintergreen essential oil, tolu balsam essential oil, patchouli essential oil, rose essential oil, palmarosa essential oil, hinoki essential oil, hiba essential oil, sandalwood essential oil, petitgrain essential oil, bay essential oil, vetiver essential oil, bergamot essential oil, Peru balsam essential oil, bois de rose essential oil, ho wood essential oil, mandarin essential oil, eucalyptus essential oil, lime essential oil, lavender essential oil, linaloe essential oil, lemongrass essential oil, lemon essential oil, rosemary essential oil, and Japanese peppermint essential oil; and others such as coffee flavors, yogurt flavors and other synthetic perfumes.

The resulting soft capsules are packaged in bottles, PTP packages, pouches, or other packages, stored, and distributed. Examples

Hereinafter, the present invention will be more specifically described with reference to examples. It will be understood that the examples are not intended to limit the present invention.

The following raw materials were used.
Iota carrageenan: A product (manufactured by MSC Co., Ltd.) containing 40% by mass of a standardizing agent (sucrose)
Kappa carrageenan: CT-1000 (manufactured by Mitsubishi Shoji Foodtech Co., Ltd.)
Waxy corn starch having undergone a heat-moisture treatment in the presence of a salt: Soft Starch SF-930 (manufactured by Sanwa Starch Co., Ltd.)*
Oxidized starch: STABILOSE (manufactured by Matsutani Chemical Industry Co., Ltd.)
Waxy potato starch: Eliane Gel 100 (manufactured by Avebe)
Glycerin: Food additive grade (manufactured by Sakamoto Yakuhin Kogyo Co., Ltd.)
* The starch paste solution had a viscosity of 312.5 mPa·s, which was the average of two measurements (B-type viscometer, starch content 40% by mass, liquid temperature 90°C).

Test Examples 1 to 11: Production of soft capsules using rotary die soft capsule filling machine
(1) Iota carrageenan, kappa carrageenan, waxy corn starch having undergone a heat-moisture treatment in the presence of a salt (which is simply referred to as "heat-moisture-treated starch" in the table below, and hereinafter the same applies), oxidized starch, and glycerin were used in the amounts (parts by mass) shown in Table 1, stirred and dispersed in water. Subsequently, they were dissolved under stirring at 90 to 98 °C and then defoamed under vacuum.
   Soft capsules were produced from each resulting shell solution using a rotary die soft capsule filling machine. Firstly, using a casting apparatus, the shell solution was cast on rotary drums at 45°C to form shell sheets. At this stage, the temperature of the shell sheets was 45°C. At this stage, [a. evaluation of strength of shell sheets] and [b. evaluation of elongation of shell sheets] were performed as described below.
(2) Subsequently, the resulting two shell sheets were each fed between a pair of cylindrical rotary dies via a lubrication roller and a deflector roll, and subjected to encapsulation while the segments were allowed to remain at room temperature (22 to 28°C) without heating, to thereby form oval (football-shaped) soft capsules. The temperature of the shell sheets in the vicinity of the rotary dies was about 29°C. At this stage, [c. evaluation of adhesive property of shell sheets immediately after capsule formation] was performed.
(3) In addition, the resulting soft capsules were stored for 24 hours in a desiccator where the relative humidity was adjusted to 20% or less, so that dried soft capsules were obtained. At this stage, [d. evaluation of elasticity of dried capsules], and [e. evaluation of stickiness of dried capsules], and [f. evaluation of transparency of dried capsules] were performed.

Also, [g. evaluation of disintegrability of dried capsules] was performed as described below with respect to Test Examples 1 to 10 where the encapsulation was successful. Table 1 shows the results.

### [a. Evaluation of strength of shell sheets]

Five expert panelists performed sensory evaluation of the strength of the shell sheets according to the following evaluation criteria.
5: Very strong
4: Strong
3: Slightly weak
2: Weak
1: Very weak

### [b. Evaluation of elongation of shell sheets]

At the same time as the evaluation of the shell sheet strength, five expert panelists performed sensory evaluation of the elongation of the shell sheets according to the following evaluation criteria.
5: Highly elongated and elastic
4: Elongate and elastic
3: Elongate but slightly less elastic
2: Hardly elongated and less elastic
1: Neither elongated nor elastic

### [c. Evaluation of adhesive property of shell sheets immediately after capsule formation]

Five expert panelists pressed the undried capsules with fingers and evaluated the adhesive property of the shell sheets immediately after the capsule formation according to the following evaluation criteria.
5: The content liquid did not leak out at all even when the capsules were strongly pressed, and leakage did not occur from any of the capsules even after standing for 24 hours.
4: The content liquid did not leak out even when the capsules were strongly pressed, but leakage occurred from some of the capsules after standing for 24 hours.
3: A very small amount of the content liquid leaked out when the capsules were strongly pressed.
2: A small amount of the content liquid leaked out even when the capsules were weakly pressed.
1: The content liquid leaked out even when the capsules were weakly pressed.

### [d. Evaluation of elasticity of dried capsules]

Five expert panelists pressed the capsules with fingers and observed their deformation to evaluate the elasticity of the capsules according to the following evaluation criteria.
5: The capsules did not deform, or the capsules quickly returned to their original shape even when they slightly deformed.
4: When pressed, the capsules slightly deformed but returned to their original shape after standing for a while.
3: When pressed, the capsules deformed and took time to return to their original shape.
2: When pressed, the capsules deformed and hardly returned to their original shape.
1: When pressed, the capsules remained deformed and did not return to their original shape.

### [e. Evaluation of stickiness of dried capsules]

Five expert panelists placed the capsules in a glass bottle, then turned the bottle upside down to observe whether the capsules fell out of the bottle, and evaluated the stickiness of the capsules according to the following evaluation criteria.
5: The capsules fell out separately when the glass bottle was simply turned upside down.
4: The capsules fell out when the glass bottle was turned upside down and shaken lightly.
3: The capsules fell out when the glass bottle was turned upside down and tapped lightly.
2: The capsules finally fell out after the glass bottle was turned upside down and tapped strongly.
1: The capsules strongly stuck to the glass bottle.

### [f. Evaluation of transparency of dried capsules]

Five expert panelists visually evaluated the transparency of the capsules according to the following evaluation criteria.
5: As transparent as gelatin shell
4: Almost as transparent as gelatin shell
3: Slightly less transparent than gelatin shell
2: Similar to the transparency of obscured glass
1: Equal to the transparency of obscured glass

### [g. Evaluation of disintegrability of dried capsules]

The time (minutes) taken for oil droplets (content) to leak out of the soft capsules was measured by the disintegration test (with disk) according to the Japanese Pharmacopoeia. Each measurement was performed on six samples (n = 6), and the average of the measurements was recorded.

**[Table 1]**

| | | Example | | | | | | | Comparative Example | | | |
|---|---|---|---|---|---|---|---|---|---|---|---|---|
| Test Example | | 1 | 2 | 3 | 4 | 5 | 6 | 7 | 8 | 9 | 10 | 11 |
| Formulation | (A) Iota carrageenan | 29.9 | 29.9 | 29.9 | 29.9 | 39.9 | 22.9 | 47.4 | 29.9 | 19.9 | 19.9 | 52.3 |
| | (B) Heat-moisture-treated starch | 10 | 40 | 60 | 70 | 34.3 | 44 | 30 | 0 | 80 | 45.7 | 27.2 |
| | Kappa carrageenan | 0.1 | 0.1 | 0.1 | 0.1 | 0.1 | 0.1 | 0.1 | 0.1 | 0.1 | 0.1 | 0.1 |
| | Oxidized starch | 60 | 30 | 10 | 0 | 25.7 | 33 | 22.5 | 70 | 0 | 34.3 | 20.4 |
| | Glycerin | 50 | 50 | 50 | 50 | 50 | 50 | 50 | 50 | 50 | 50 | 50 |
| | Purified water | 155 | 155 | 155 | 155 | 220 | 130 | 270 | 150 | 130 | 130 | 300 |
| | [(A)/(B)] | 2.99 | 0.75 | 0.50 | 0.43 | 1.16 | 0.52 | 1.58 | - | 0.25 | 0.44 | 1.92 |
| | [Carrageenan /starch] | 0.43 | 0.43 | 0.43 | 0.43 | 0.67 | 0.30 | 0.90 | 0.43 | 0.25 | 0.25 | 1.10 |
| Evaluation of shell sheets | a. Shell sheet strength | 4 | 5 | 4 | 4 | 5 | 4 | 4 | 4 | 3 | 3 | 2 |
| | b. Elongation | 4 | 4 | 4 | 4 | 4 | 4 | 4 | 4 | 4 | 3 | 3 |
| | c. Adhesive property immediately after capsule formation | 4 | 5 | 5 | 5 | 5 | 4 | 4 | 3 | 4 | 4 | - |
| Whether encapsulation was possible or not | | Possible | Possible | Possible | Possible | Possible | Possible | Possible | Possible | Possible | Possible | Impossible |
| Evaluation of dried capsules | d. Elasticity | 5 | 5 | 4 | 4 | 5 | 4 | 5 | 5 | 4 | 4 | - |
| | e. Stickiness | 4 | 5 | 4 | 4 | 5 | 4 | 5 | 3 | 4 | 4 | - |
| | f. Transparency | 4 | 5 | 4 | 4 | 5 | 4 | 4 | 3 | 4 | 4 | - |
| | g. Disintegrability (rupturing time (minutes)) | 3.0 | 3.0 | 3.0 | 3.0 | 3.0 | 4.0 | 3.0 | 3.0 | 4.0 | 5.0 | - |

Table 1 shows that the strength and adhesive property of the shell sheets on the capsule formation were good in Test Examples 1 to 7 where iota carrageenan and heat-moisture-treated starch were used in a specific ratio. After the encapsulation, the resulting soft capsules also had good shell performance and good quality in Test Examples 1 to 7.

In contrast, the strength and adhesive property of the shell sheets were relatively inferior in Test Example 8 where iota carrageenan and oxidized starch were used and in Test Examples 9 and 10 where the content ratio of carrageenan to starch was relatively low. In Test Example 11, encapsulation was not possible due to low adhesive property.

### Test Examples 12 and 13 : Production of soft capsules using rotary die soft capsule filling machine

Iota carrageenan, kappa carrageenan, waxy corn starch having undergone a heat-moisture treatment in the presence of a salt, oxidized starch, and glycerin were used in the amounts (parts by mass) shown in Table 2, and stirred and dispersed in water. Shell solutions were each prepared as in the above test example and then subjected to formation of self-cut shaped soft capsules using a rotary die soft capsule filling machine.

As in the above test example, [a. evaluation of strength of the shell sheets], [b. evaluation of elongation of shell sheets], [c. evaluation of adhesive property of shell sheets immediately after the capsule formation], [d. evaluation of elasticity of dried capsules], [e. evaluation of stickiness of dried capsules], and [f. evaluation of transparency of dried capsules] were also performed. Table 2 shows the results.

**[Table 2]**

| | | Example | Comparative Example |
|---|---|---|---|
| Test Example | | 12 | 13 |
| Formulation | (A) Iota carrageenan | 28 | 28 |
| | (B) Heat-moisture-treated starch | 30 | 0 |
| | Kappa carrageenan | 2 | 2 |
| | Oxidized starch | 40 | 70 |
| | Glycerin | 45 | 45 |
| | Purified water | 150 | 150 |
| | [(A)/(B)] | 0.93 | - |
| | [Carrageenan/starch] | 0.43 | 0.43 |
| Evaluation of shell sheets | a. Shell sheet strength | 4 | 4 |
| | b. Elongation | 4 | 4 |
| | c. Adhesive property immediately after capsule formation | 5 | 3 |
| Whether encapsulation was possible or not | | Possible | Possible |
| Evaluation of dried capsules | d. Elasticity | 4 | 4 |
| | e. Stickiness | 4 | 4 |
| | f. Transparency | 5 | 3 |
| | g. Disintegrability (rupturing time (minutes)) | - | - |

Table 2 shows that the strength and adhesive property of the shell sheets on the capsule formation were good in Test Example 12 where iota carrageenan and heat-moisture-treated starch were used together and that the resulting soft capsules also had good shell performance after the encapsulation in Test Example 12.

In contrast, leakage of the content due to low adhesive property was observed in Test Example 13 where iota carrageenan and oxidized starch were used.

### Test Example 14: Production of soft capsules using rotary die soft capsule filling machine

Iota carrageenan, waxy corn starch having undergone a heat-moisture treatment in the presence of a salt, waxy potato starch, and glycerin were used in the amounts (parts by mass) shown in Table 3, and stirred and dispersed in water. A shell solution was prepared as in the above test example and then subjected to formation of self-cut shaped soft capsules using a rotary die soft capsule filling machine.

As in the above test example, [a. evaluation of strength of shell sheets], [b. evaluation of elongation of shell sheets], [c. evaluation of adhesive property of shell sheets immediately after capsule formation], [d. evaluation of elasticity of dried capsules], [e. evaluation of stickiness of dried capsules], and [f. evaluation of transparency of dried capsules] were also performed. Table 3 shows the results.

**[Table 3]**

| | | Example |
|---|---|---|
| Test Example | | 14 |
| Formulation | (A) Iota carrageenan | 30 |
| | (B) Heat-moisture-treated starch | 45 |
| | Waxy starch | 25 |
| | Glycerin | 50 |
| | Purified water | 160 |
| | [(A)/(B)] | 0.67 |
| | [Carrageenan/starch] | 0.43 |
| Evaluation of shell sheets | a. shell sheet strength | 5 |
| | b. Elongation | 4 |
| | c. Adhesive property immediately after capsule formation | 5 |
| Whether encapsulation was possible or not | | Possible |
| Evaluation of dried capsules | d. Elasticity | 5 |
| | e. Stickiness | 5 |
| | f. Transparency | 5 |
| | g. Disintegrability (rupturing time (minutes)) | - |

Table 3 shows that the strength and adhesive property of the shell sheets on the capsule formation were good in Test Example 14 where iota carrageenan, heat-moisture-treated starch, and waxy potato starch were used together and that the resulting soft capsules also had good shell performance, particularly high transparency, after the encapsulation in Test Example 14.

## Claims

1. A soft capsule shell comprising:
(A) iota carrageenan; and
(B) waxy corn starch having undergone a heat-moisture treatment in the presence of a salt,
the soft capsule shell having a content mass ratio [(A)/(B)] of the component (A) to the component (B) of 0.4 to 3,
the soft capsule shell having a content mass ratio [carrageenan/starch] of carrageenan comprising the component (A) to starch comprising the component (B) of 0.29 to 1.

2. The soft capsule shell according to claim 1, further comprising a plasticizer.

3. The soft capsule shell according to claim 1 or 2, wherein the content of the iota carrageenan (A) is from 20 to 55% by mass based on the total amount of solid components.

4. The soft capsule shell according to any one of claims 1 to 3, wherein the content of the waxy corn starch having undergone a heat-moisture treatment in the presence of a salt (B) is from 10 to 80% by mass based on the total amount of solid components.

5. The soft capsule shell according to any one of claims 1 to 4, further comprising kappa carrageenan.

6. The soft capsule shell according to claim 5, wherein the content of the kappa carrageenan is from 0.05 to 3.5% by mass based on the total amount of solid components.

7. The soft capsule shell according to any one of claims 1 to 6, further comprising waxy potato starch.

8. A soft capsule obtained using the soft capsule shell according to any one of claims 1 to 7.

9. A method for producing a soft capsule, the method comprising the steps of:
preparing a soft capsule shell solution comprising (A) iota carrageenan and (B) waxy corn starch having undergone a heat-moisture treatment in the presence of a salt, wherein the solution has a content mass ratio [(A)/(B)] of the component (A) to the component (B) of 0.4 to 3 and also has a content mass ratio [carrageenan/starch] of carrageenan comprising the component (A) to a starch comprising the component (B) of 0.29 to 1;
casting the soft capsule shell solution on a pair of rotary drums to form a pair of shell sheets; and
feeding the pair of shell sheets between a pair of rotary dies, wherein
the pair of shell sheets are heated to a temperature higher than room temperature until the pair of shell sheets reach the pair of rotary dies, so that the shell sheets are pressure-sealed and formed into a capsule while segments arranged above the dies are kept at room temperature without heating.

## Patentansprüche

1. Hülle für eine Weichkapsel, umfassend:
(A) Iota-Carrageen und
(B) Wachsmaisstärke, die einer Wärme-Feucht-Behandlung in Anwesenheit eines Salzes unterzogen wurde,
wobei die Hülle für eine Weichkapsel ein Gehaltsmassenverhältnis [(A) / (B)] des Bestandteils (A) zu dem Bestandteil (B) von 0,4 bis 3 aufweist,
wobei die Hülle für eine Weichkapsel ein Gehaltsmassenverhältnis [Carrageen / Stärke] von Carrageen, das den Bestandteil (A) umfasst, zu Stärke, die den Bestandteil (B) umfasst, von 0,29 bis 1 aufweist.

2. Hülle für eine Weichkapsel nach Anspruch 1, ferner einen Weichmacher umfassend.

3. Hülle für eine Weichkapsel nach Anspruch 1 oder 2, wobei der Gehalt an Iota-Carrageen (A) von 20 bis 55 Massenprozent, basierend auf der Gesamtmenge der festen Bestandteile, beträgt.

4. Hülle für eine Weichkapsel nach einem der Ansprüche 1 bis 3, wobei der Gehalt an Wachsmaisstärke, die einer Wärme-Feucht-Behandlung in Anwesenheit eines Salzes (B) unterzogen wurde, von 10 bis 80 Massenprozent, basierend auf der Gesamtmenge der festen Bestandteile, beträgt.

5. Hülle für eine Weichkapsel nach einem der Ansprüche 1 bis 4, ferner Kappa-Carrageen umfassend.

6. Hülle für eine Weichkapsel nach Anspruch 5, wobei der Gehalt an Kappa-Carrageen von 0,05 bis 3,5 Massenprozent, basierend auf der Gesamtmenge der festen Bestandteile, beträgt.

7. Hülle für eine Weichkapsel nach einem der Ansprüche 1 bis 6, ferner Wachskartoffelstärke umfassend.

8. Weichkapsel, die unter Verwendung der Hülle für eine Weichkapsel nach einem der Ansprüche 1 bis 7 erhalten wird.

9. Verfahren zur Herstellung einer Weichkapsel, wobei das Verfahren die folgenden Schritte umfasst:
Herstellung einer Lösung der Hülle für eine Weichkapsel, umfassend (A) Iota-Carrageen und (B) Wachsmaisstärke, die einer Wärme-Feucht-Behandlung in Anwesenheit eines Salzes unterzogen wurde, wobei die Lösung ein Gehaltsmassenverhältnis [(A) / (B)] des Bestandteils (A) zu dem Bestandteil (B) von 0,4 bis 3 aufweist und auch ein Gehaltsmassenverhältnis [Carrageen / Stärke] von Carrageen, das den Bestandteil (A) umfasst, zu Stärke, die den Bestandteil (B) umfasst, von 0,29 bis 1 aufweist;
Giessen der Lösung der Hülle für eine Weichkapsel in ein Paar Drehtrommeln, um ein Paar Hüllenschalen zu bilden und
Zuführen des Paares der Hüllenschalen zwischen ein Paar rotierende Walzen, wobei das Paar der Hüllenschalen auf eine Temperatur erwärmt wird, die höher als Raumtemperatur ist, bis das Paar der Hüllenschalen das Paar der rotierenden Walzen erreicht, so dass die Hüllenschalen mit Druck versiegelt und zu Kapseln geformt werden, während die Segmente, die über den Walzen angeordnet sind, ohne Erwärmen bei Raumtemperatur gehalten werden.

## Revendications

1. Enveloppe de capsule molle, comprenant :
(A) du carraghénane iota ; et
(B) de l'amidon de maïs cireux qui a subi un traitement à la chaleur humide en présence d'un sel,
l'enveloppe de capsule molle ayant un rapport de masse de contenu [(A)/(B)] du composant (A) sur le composant (B) de 0,4 à 3,
l'enveloppe de capsule molle ayant un rapport de masse de contenu [carraghénane/amidon] du carraghénane comprenant le composant (A) sur l'amidon comprenant le composant (B) de 0,29 à 1.

2. Enveloppe de capsule molle selon la revendication 1, comprenant également un plastifiant.

3. Enveloppe de capsule molle selon la revendication 1 ou 2, dans laquelle le contenu du carraghénane iota (A) est de 20 à 55 % en masse sur la base de la quantité totale de composants solides.

4. Enveloppe de capsule molle selon l'une quelconque des revendications 1 à 3, dans laquelle le contenu de l'amidon de maïs cireux qui a subi un traitement à la chaleur humide en présence d'un sel (B) est de 10 à 80 % en masse sur la base de la quantité totale de composants solides.

5. Enveloppe de capsule molle selon l'une quelconque des revendications 1 à 4, comprenant également du carraghénane kappa.

6. Enveloppe de capsule molle selon la revendication 5, dans laquelle le contenu du carraghénane kappa (A) est de 0,05 à 3,5 % en masse sur la base de la quantité totale de composants solides.

7. Enveloppe de capsule molle selon l'une quelconque des revendications 1 à 6, comprenant également de l'amidon de pomme de terre cireuse.

8. Capsule molle obtenue à l'aide de l'enveloppe de capsule molle selon l'une quelconque des revendications 1 à 7.

9. Procédé pour produire une capsule molle, le procédé comprenant les étapes :
de préparation d'une solution pour enveloppe de capsule molle comprenant (A) du carraghénane iota et (B) de l'amidon de maïs cireux qui a subi un traitement à la chaleur humide en présence d'un sel, la solution ayant un rapport de masse de contenu [(A)/(B)] du composant (A) sur le composant (B) de 0,4 à 3 ainsi qu'un rapport de masse de contenu [carraghénane/amidon] du carraghénane comprenant le composant (A) sur l'amidon comprenant le composant (B) de 0,29 à 1 ;
de coulage de la solution pour enveloppe de capsule molle sur deux tambours rotatifs afin de former deux feuilles d'enveloppe ; et
d'amenée des deux feuilles d'enveloppe entre deux matrices rotatives,
les deux feuilles d'enveloppe étant portées à une température supérieure à la température ambiante jusqu'à ce qu'elles atteignent les deux matrices rotatives, de sorte qu'elles sont scellées sous pression pour prendre la forme d'une capsule, tandis que des segments disposés au-dessus des matrices sont maintenus à température ambiante, sans chauffage.
